# EUROPEAN PATENT SPECIFICATION

(11) **EP 3 330 295 B1**
(45) Date of publication and mention of the grant of the patent: **21.08.2019**
(21) Application number: 16830360.0
(22) Date of filing: 15.07.2016
(51) Int. Cl.: C08F 2/50, C08F 20/18, C08F 20/56, C08F 222/38, C08K 5/06, C09D 4/00, C09J 4/00, C08K 5/07, C08F 2/48

(54) **PHOTOPOLYMERIZATION INITIATOR COMPOSITION, ACTIVE ENERGY RAY-CURABLE COMPOSITION, AND CURED PRODUCT**
PHOTOPOLYMERISIERUNGSINITIATORZUSAMMENSETZUNG, DURCH AKTIVE ENERGIESTRAHLUNG HÄRTBARE ZUSAMMENSETZUNG UND GEHÄRTETES PRODUKT
COMPOSITION D'INITIATEUR DE PHOTOPOLYMÉRISATION, COMPOSITION DURCISSABLE PAR UN RAYONNEMENT D'ÉNERGIE ACTIVE ET PRODUIT DURCI

(30) Priority: 28.07.2015 JP 2015148547
(43) Date of publication of application: 06.06.2018
(73) Proprietor: FUJIFILM Corporation, Tokyo 106-8620 (JP)
(72) Inventor: YAMASHITA Masako, Ashigara-kami-gun Kanagawa 258-8577 (JP)
(74) Representative: Hoffmann Eitle
(86) International application number: PCT/JP2016/071052
(87) International publication number: WO 2017/018255

(56) References cited:
- WO-A1-2016/052140
- JP-A- H10 330 317
- JP-A- 2007 137 964
- JP-A- 2008 247 939
- JP-A- 2015 010 196
- JP-A- 2015 025 076

## Description

### BACKGROUND OF THE INVENTION

### 1. Field of the Invention

The present invention relates to a photopolymerization initiator composition, an active energy ray-curable composition, and a cured product.

### 2. Description of the Related Art

In the related art, an active energy ray-curable composition containing a photopolymerization initiator and a polymerizable compound is known, as disclosed for example in JP2015-25076, JP2007-13796 and JP2015-10196. The active energy ray-curable composition is used for a variety of uses. Examples of the uses include paints, adhesives, pressure sensitive adhesives, inks, coatings, functional membranes, films, optical materials, printing plate materials, semiconductor materials, recording materials, paper additives, medical materials, plastics, functional gels.

Among the components contained in the active energy ray-curable composition, the photopolymerization initiator causes the polymerization of the polymerizable compound by the irradiation of active energy rays. In this way, the active energy ray-curable composition is cured, and a cured product (cured film) is obtained.

As such a photopolymerization initiator, for example, Claim 2 in JP1987-502403A (JP-S62-502403A) discloses 4-(2-hydroxydiethoxy)-phenyl 2-hydroxy-2-propylketone.

### SUMMARY OF THE INVENTION

Depending on the type of the photopolymerization initiator, the storage stability of the photopolymerization initiator composition exhibited in a case where the composition is stored under a low-temperature condition (-20°C) for a predetermined period of time (hereinafter, simply described as "storage stability at a low temperature" as well) deteriorates, and sometimes the components derived from the photopolymerization initiator are precipitated. In a case where such precipitates are generated, the deterioration of handleability such as the deterioration of solubility with other components may occur.

Furthermore, in a case where a cured product (cured film) is formed by curing the active energy ray-curable composition, sometimes the precipitates derived from the components contained in the active energy ray-curable composition are generated on the surface of the cured product (bleed out). This phenomenon particularly markedly occurs in a case where a thermocycling test is performed in which the storage of the cured film at a high temperature (60°C) and a low temperature (-20°C) is repeated as one cycle. In a case where the bleed out occurs, the transparency of the cured film may deteriorate.

Therefore, an object of the present invention is to provide a photopolymerization initiator composition which exhibits excellent storage stability at a low temperature and has an excellent effect of inhibiting bleed out occurring on the surface of a cured product after the thermocycling test. Another object of the present invention is to provide an active energy ray-curable composition and a cured product formed using the composition.

Regarding the aforementioned objects, the inventor of the present invention conducted a thorough examination. As a result, the inventor has found that desired effects are obtained using a photopolymerization initiator composition containing a predetermined photopolymerization initiator and a predetermined additive, and accomplished the present invention.

That is, the inventor of the present invention has found that the aforementioned objects can be achieved by the following constitution.
[1] A photopolymerization initiator composition comprising a photopolymerization initiator represented by General Formula (I) which will be described later, and at least one kind of additive selected from the group consisting of compounds represented by General Formulae (II) to (IV) which will be described later, in which in General Formulae (I) to (IV) which will be described later, V¹, V², V³, and V⁴ each independently represent a hydrogen atom or a substituent as defined below, and in General Formula (I) which will be described later, n represents an integer of 1 to 5.
[2] The photopolymerization initiator composition described in [1], in which n in General Formulae (I) to (IV) is 1.
[3] The photopolymerization initiator composition described in [1] or [2], in which a content of the additive with respect to 100 parts by mass of the photopolymerization initiator is 0.0005 to 10 parts by mass.
[4] The photopolymerization initiator composition described in any one of [1] to [3], in which the additive is at least one kind of compound selected from the group consisting of compounds represented by General Formula (III) and General Formula (IV).
[5] An active energy ray-curable composition comprising the photopolymerization initiator composition described in any one of [1] to [4] and a polymerizable compound.
[6] The active energy ray-curable composition described in [5], in which a content of the photopolymerization initiator composition with respect to 100 parts by mass of the polymerizable compound is 0.1 to 10 parts by mass.
[7] A cured product obtained by curing the active energy ray-curable composition described in [5] or [6].

As will be described below, according to the present invention, it is possible to provide a photopolymerization initiator composition which exhibits excellent storage stability at a low temperature and has an excellent effect of inhibiting bleed out occurring on the surface of a cured product after the thermocycling test. Furthermore, the present invention can provide an active energy ray-curable composition and a cured product formed using the composition.

### DESCRIPTION OF THE PREFERRED EMBODIMENTS

Hereinafter, the photopolymerization initiator composition, the active energy ray-curable composition, and the cured product of the present invention will be described. The present invention is not limited to the embodiments descried below, and includes various modification examples embodied within a scope in which the gist of the present invention is not changed.

In the present specification, in a case where there is no description regarding whether a group (atomic group) is a substituted or unsubstituted, the group includes both a group not having a substituent and a group having a substituent. For example, "alkyl group" includes not only an alkyl group not having a substituent (unsubstituted alkyl group) but also an alkyl group having a substituent (substituted alkyl group).

In the present specification, "active energy rays", "actinic rays", or "radiation" means the rays including, for example, visible rays, ultraviolet rays, far-ultraviolet rays, electron beams, α-rays, γ-rays, X-rays, and the like. Furthermore, in the present specification, in a case where simply "light" is mentioned, the term means active energy rays, actinic rays, or radiation.

In the present specification, unless otherwise specified, "exposure" means not only the exposure performed using a mercury lamp, a high-pressure mercury lamp, a metal halide lamp, an ultraviolet fluorescent lamp, a light emitting diode (LED), ultraviolet rays from a light source such as a laser light source, far-ultraviolet rays represented by an excimer laser, X-rays, extreme ultraviolet (EUV) light, but also the drawing performed using particle beams such as electron beams and ion beams.

In the present specification, "(meth)acrylate" represents acrylate and methacrylate, "(meth)acryl" represents acryl and methacryl, and "(meth)acryloyl" represents acryloyl and methacryloyl.

In the present specification, "unit molecule" has the same definition as "monomer". In the present specification, "monomer" refers to a compound which is distinguished from an oligomer and a polymer, and has a weight-average molecular weight equal to or smaller than 2,000.

In the present specification, "polymerizable compound" refers to a compound having a polymerizable group and may be a monomer or a polymer. The polymerizable group refers to a group involved in a polymerization reaction.

In the present specification, a range of numerical values described using "to" means a range including the numerical values described before and after "to" as a lower limit and an upper limit respectively.

### [Photopolymerization initiator composition]

The photopolymerization initiator composition of the present invention contains a photopolymerization initiator represented by General Formula (I), which will be described later, and at least one kind of additive selected from the group consisting of compounds represented by General Formulae (II) to (IV) which will be described later.

Hereinbelow, in the present specification, the photopolymerization initiator represented by General Formula (I) which will be described later will be referred to as "specific photopolymerization initiator". Furthermore, the at least one kind of additive selected from the group consisting of compounds represented by General Formulae (II) to (IV) which will be described later will be referred to as "specific additive".

The photopolymerization initiator composition of the present invention contains the specific photopolymerization initiator and the specific additive. Therefore, the composition exhibits excellent storage stability at a low temperature and has an excellent effect of inhibiting bleed out occurring on the surface of a cured product after the thermocycling test.

The reason why the composition has the aforementioned properties has not yet been clarified, but is assumed to be as below.

In a case where the specific photopolymerization initiator is stored at a low temperature, the crystallization of the specific photopolymerization initiator tends to be accelerated. It is considered that at this time, in a case where the specific photopolymerization initiator coexists with the specific additive, the specific additive functions as an impurity of the specific photopolymerization initiator, and hence the crystallization of the specific photopolymerization initiator could be inhibited. Presumably, for this reason, the photopolymerization initiator composition may exhibit excellent storage stability at a low temperature.

Furthermore, presumably, because both the specific photopolymerization initiator and the specific additive contains a polyethylene oxide group, the initiator and the additive may function as a surfactant in an active energy ray-curable composition (hereinafter, simply referred to as "curable composition" as well). It is considered that for this reason, even in a case where the composition was under severe conditions such as the thermocycling test, it is possible to inhibit the occurrence of a precipitate on the surface of a cured product obtained by curing the curable composition.

In addition, because the specific additive has a structure derived from the specific photopolymerization initiator (specifically, the specific additive includes a multimeric skeleton of the specific photopolymerization initiator), the structure thereof is similar to the structure of the specific photopolymerization initiator. It is considered that accordingly, the specific additive may be excellently compatible with the component (for example, the unreacted specific photopolymerization initiator or the decomposition product of the specific photopolymerization initiator) derived from the specific photopolymerization initiator included in the cured product. Presumably, for this reason, even in a case where the composition was under severe conditions such as the thermocycling test, it is possible to inhibit the component derived from the specific photopolymerization initiator from being precipitated on the surface of the cured product.

It is considered that in this way, the effect of inhibiting bleed out occurring on the surface of the cured product after the thermocycling test is improved.

### <Specific photopolymerization initiator>

The photopolymerization initiator composition of the present invention contains a photopolymerization initiator (specific photopolymerization initiator) represented by General Formula (I). The specific photopolymerization initiator has a function of initiating the polymerization reaction of a polymerizable compound, which will be described later, by the irradiation of active energy rays.

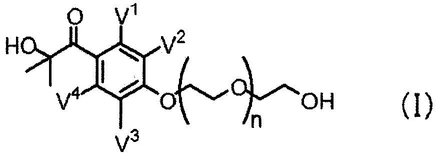

In General Formula (I), V¹, V², V³, and V⁴ each independently represent a hydrogen atom or a substituent. In General Formula (I), n represents an integer of 1 to 5.

In a case where n in General Formula (I) is equal to or greater than 1, the storage stability of the photopolymerization initiator composition at a low temperature is improved, and the occurrence of bleed out on the surface of a cured product after the thermocycling test is inhibited. In a case where n is equal to or smaller than 5, the solubility of the photopolymerization initiator composition in water is improved, and the sensitivity of the curable composition is kept high.

In view of making the aforementioned effects more strongly exhibited, n is preferably 1 to 3, more preferably 1 or 2, and even more preferably 1.

In contrast, in a case where n is less than 1 (that is, n = 0), the stability of the components contained in the active energy ray-curable composition tends to deteriorate. Consequently, the storage stability of the photopolymerization initiator composition at a low temperature deteriorates, or the bleed out easily occurs on the surface of a cured product after the thermocycling test.

In a case where n is greater than 5, the solubility of the photopolymerization initiator composition in water deteriorates, or the sensitivity of the curable composition deteriorates.

The substituent represented by V¹ to V⁴ is selected from a halogen atom, an alkyl group, an alkoxy group, a hydroxyl group, an alkylthio group, a mercapto group, an acyl group, and an amino group.

As the halogen atom represented by V¹ to V⁴, a fluorine atom, a chlorine atom, a bromine atom, and an iodine atom are preferable, a chlorine atom and a bromine atom are more preferable, and a chlorine atom is particularly preferable.

The number of carbon atoms in the alkyl group represented by V¹ to V⁴ is 1 to 6, and preferably 1 to 3.

The alkyl group represented by V¹ to V⁴ is preferably a linear alkyl group or an alkyl group having a branched chain. Furthermore, the alkyl group may have an alicyclic structure.

Examples of the alkyl group represented by V¹ to V⁴ include a methyl group, an ethyl group, a n-propyl group, an isopropyl group, a n-butyl group, a sec-butyl group, a tert-butyl group, a n-pentyl group, a n-hexyl group, and a cyclohexyl group. Among these, a methyl group, an ethyl group, a n-propyl group, and an isopropyl group are preferable.

The number of carbon atoms in the alkoxy group represented by V¹ to V⁴ is 1 to 6, and preferably 1 to 3.

The alkoxy group represented by V¹ to V⁴ is preferably a linear alkoxy group or an alkoxy group having a branched chain. Furthermore, the alkoxy group may have an alicyclic structure.

Examples of the alkoxy group represented by V¹ to V⁴ include a methoxy group, an ethoxy group, a n-propyloxy group, an isopropyloxy group, a n-butoxy group, a sec-butoxy group, a tert-butoxy group, a n-pentyloxy group, a n-hexyloxy group, and a cyclohexyloxy group. Among these, a methoxy group, an ethoxy group, a n-propyloxy group, and an isopropyloxy group are preferable.

The number of carbon atoms in the alkylthio group represented by V¹ to V⁴ is 1 to 6, and preferably 1 to 4.

The alkylthio group represented by V¹ to V⁴ may be a linear alkylthio group or an alkylthio group having a branched chain. Furthermore, the alkylthio group may have an alicyclic structure.

Examples of the alkylthio group represented by V¹ to V⁴ include a methylthio group, an ethylthio group, a n-propylthio group, an isopropylthio group, a n-butylthio group, a sec-butylthio group, a tert-butylthio group, a n-pentylthio group, a n-hexylthio group, and a cyclohexylthio group. Among these, a methylthio group, an ethylthio group, a n-propylthio group, and an isopropylthio group are preferable.

The number of carbon atoms in the acyl group represented by V¹ to V⁴ is 1 to 6, and preferably 1 to 3.

The acyl group represented by V¹ to V⁴ is preferably a linear acyl group or an acyl group having a branched chain.

Examples of the acyl group represented by V¹ to V⁴ include a formyl group, an acetyl group, an ethylacyl group, a n-propylacyl group, and an isopropylacyl group. Among these, a formyl group, an acetyl group, and an ethylacyl group are preferable.

Examples of the amino group represented by V¹ to V⁴ include -NH₂, an alkylamino group (a monoalkylamino group (-NR^{V1}) and a dialkylamino group (-NR^{V1}R^{V2})). Among these, an alkylamino group is preferable, and a dialkylamino group is more preferable. R^{V1} and R^{V2} each independently represent an alkyl group (preferably having 1 to 6 carbon atoms and more preferably having 1 to 3 carbon atoms).

V¹ to V⁴ each independently preferably represent a hydrogen atom, a halogen atom, an alkyl group, an alkoxy group, and an alkylthio group, more preferably represent a hydrogen atom, an alkoxy group, and an alkylthio group, and even more preferably represent a hydrogen atom.

As a more preferable aspect of the specific photopolymerization initiator, an aspect can be exemplified in which two or more (preferably three or more, and most preferably four) among V¹ to V⁴ represent a hydrogen atom.

Specific examples (example compounds) of the specific photopolymerization initiator will be shown below, but the specific photopolymerization initiator is not limited thereto.

**[Table 1]**

| Example compound | General Formula (I) | | | | |
|---|---|---|---|---|---|
| | n | V¹ | V² | V³ | V⁴ |
| (I)-1 | 1 | H | H | H | H |
| (I)-2 | 2 | H | H | H | H |
| (I)-3 | 3 | H | H | H | H |
| (I)-4 | 5 | H | H | H | H |
| (I)-5 | 1 | CH₃ | H | H | H |
| (I)-6 | 1 | H | CH₃ | H | H |
| (I)-7 | 1 | H | OCH₃ | H | H |
| (I)-8 | 1 | H | Cl | H | H |
| (1)-9 | 1 | H | Br | H | H |
| (I)-10 | 1 | H | OH | H | H |
| (I)-11 | 1 | Cl | Cl | H | H |
| (I)-12 | 1 | SCH₃ | H | H | H |
| (I)-13 | 2 | Cl | H | Cl | H |
| (I)-14 | 2 | H | CH₃ | H | H |
| (I)-15 | 2 | H | N(CH₃)₂ | Cl | H |
| (I)-16 | 2 | CH₃ | CH₃ | H | H |
| (I)-17 | 3 | H | OH | H | H |
| (I)-18 | 3 | H | CH₃ | H | H |
| (I)-19 | 5 | H | COCH₃ | H | H |
| (I)-20 | 5 | H | CH₃ | H | H |

The specific photopolymerization initiator can be synthesized based on the methods described in paragraphs "0067" to "0071" and paragraphs "0112" to "0115" in JP2000-186242A, for example. Furthermore, the specific photopolymerization initiator can be synthesized by the method described in Examples which will be described later.

The molecular weight of the specific photopolymerization initiator is preferably within a range of 260 to 450, and more preferably within a range of 260 to 360, because then the dispersibility of the specific photopolymerization initiator in the curable composition (which will be described later) is improved, and hence the function thereof as a photopolymerization initiator is further improved.

The photopolymerization initiator composition of the present invention may contain a polymerization initiator other than the specific photopolymerization initiator. Examples of such a polymerization initiator include a thermal polymerization initiator, a photopolymerization initiator other than the specific photopolymerization initiator, and the like.

As the thermal polymerization initiator, known compounds can be used without restriction. For example, it is possible to use the thermal radical polymerization initiators described in paragraphs "0130" to "0132" in JP2014-80570A.

As the photopolymerization initiator, known compounds can be used without restriction. For example, it is possible to use the photo-radical photopolymerization initiators described in paragraphs "0133" to "0148" in JP2014-80570A.

### <Specific additive>

The photopolymerization initiator composition of the present invention contains the specific additive. The specific additive of the present invention has a structure derived from the aforementioned specific photopolymerization initiator (specifically, the specific additive includes a multimeric skeleton of the specific photopolymerization initiator), and includes at least one of a dimeric skeleton of the specific photopolymerization initiator or a trimeric skeleton of the specific photopolymerization initiator. Specifically, the specific additive of the present invention is at least one kind of additive selected from the group consisting of compounds represented by General Formulae (II) to (IV).

One kind of the specific additive may be used singly, or two or more kinds thereof may be used in combination.

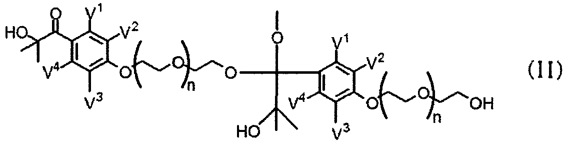

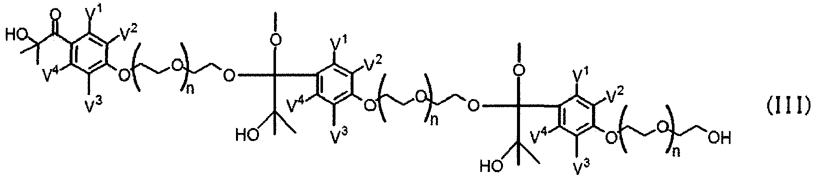

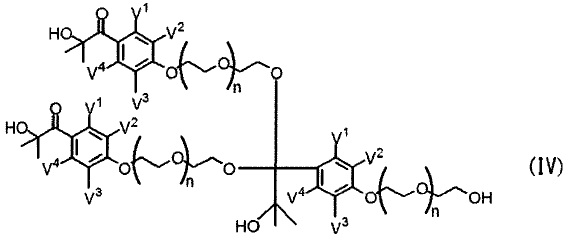

V¹ to V⁴ in General Formulae (II) to (IV) have the same definition as V¹ to V⁴ in General Formula (I), and the preferable aspect thereof is also the same. Furthermore, n in General Formulae (II) to (IV) has the same definition as n in General Formula (I), and the preferable aspect thereof is also the same.

A plurality of V¹'s in General Formulae (II) to (IV) may be the same as or different from each other. Similarly to the plurality of V¹'s, a plurality of V²'s, a plurality of V³'s, a plurality of V⁴'s, and a plurality of n's may be the same as or different from each other.

The compound represented by General Formula (II) includes a dimeric skeleton of the specific photopolymerization initiator. Furthermore, the compounds represented by General Formulae (III) and (IV) have a trimeric skeleton of the specific photopolymerization initiator.

Among the compounds represented by General Formulae (II) to (IV), at least one kind of compound selected from the group consisting of compounds represented by General Formula (III) and General Formula (IV) is preferable as the specific additive, and the compound represented by General Formula (IV) is more preferable as the specific additive. In a case where such a compound is used, the occurrence of bleed out on a cured product after the thermocycling test is further inhibited.

The content of the specific additive with respect to 100 parts by mass of the specific photopolymerization initiator is preferably 0.0005 to 10 parts by mass, more preferably 0.001 to 8 parts by mass, and particularly preferably 0.005 to 5 parts by mass.

In a case where the content of the specific additive is equal to or greater than 0.0005 parts by mass, the storage stability of the photopolymerization initiator composition at a low temperature is further improved, and the occurrence of bleed out on the surface of a cured product after the thermocycling test is further inhibited.

In a case where the content of the specific additive is equal to or smaller than 10 parts by mass, the solubility of the photopolymerization initiator composition in water is improved, and the sensitivity of the curable composition is improved.

The photopolymerization initiator composition of the present invention may further contain, in addition to the specific additive, an additive including a multimeric skeleton of the specific photopolymerization initiator. Examples of the additive including a multimeric skeleton of the specific photopolymerization initiator other than the specific additive include compounds having a skeleton composed of four or more molecules of the specific photopolymerization initiator.

The method for manufacturing the specific additive is not particularly limited, and can be performed using the aforementioned specific photopolymerization initiator, for example. Specifically, the specific additive can be synthesized by the method described in examples which will be described later.

### <Preparation method>

The photopolymerization initiator composition of the present invention is obtained by mixing the aforementioned components together and stirring the mixture, and the preparation method of the composition is not particularly limited.

### [Curable composition]

The curable composition (active energy ray-curable composition) of the present invention contains the aforementioned photopolymerization initiator composition and a polymerizable compound (which will be described later). The curable composition of the present invention contains the photopolymerization initiator composition. Therefore, the curable composition exhibits excellent sensitivity with respect to active energy rays.

### <Photopolymerization initiator composition>

The photopolymerization initiator composition contained in the curable composition of the present invention is as described above.

The content of the photopolymerization initiator composition with respect to 100 parts by mass of the polymerizable compound is preferably 0.1 to 10 parts by mass, more preferably 0.2 to 9 parts by mass, and particularly preferably 0.3 to 8 parts by mass.

In a case where the content of the photopolymerization initiator composition is equal to or greater than 0.1 parts by mass, the sensitivity of the curable composition is further improved. In a case where the content of the photopolymerization initiator composition is equal to or smaller than 10 parts by mass, the occurrence of bleed out on the surface of a cured product after the thermocycling test is further inhibited.

### <Polymerizable compound>

The curable composition of the present invention contains a polymerizable compound. The polymerizable compound is preferably a radically polymerizable monomer. Typically, the radically polymerizable monomer has a radically polymerizable group. The radically polymerizable group is a group which can be polymerized by the action of a radical.

One kind of the polymerizable compound may be used singly, or two or more kinds thereof may be used in combination.

Specifically, the radically polymerizable monomer is selected from compounds having at least one radically polymerizable group and preferably having two or more radically polymerizable groups. From the viewpoint of excellent reactivity, compounds having two to six radically polymerizable groups, that is, polyfunctional polymerizable compounds are more preferable. A group of such compounds is well known in the field of the related art. In the present invention, these compounds can be used without particular restriction. These may be in any chemical form such as a monomer or a prepolymer which is in other word a dimer, a trimer, or an oligomer, a mixture of these, and a multimer of these, for example.

The radically polymerizable group is preferably an ethylenically unsaturated group. As the ethylenically unsaturated group, a styryl group, a (meth)acryloyl group, a (meth)acryloyloxy group, and an allyl group are preferable, a (meth)acryloyl group and a (meth)acryloyloxy group are more preferable, and a (meth)acryloyloxy group is even more preferable.

More specifically, examples of the monomer and the prepolymer thereof include an unsaturated carboxylic acid (for example, acrylic acid, methacrylic acid, itaconic acid, crotonic acid, isocrotonic acid, maleic acid) or esters and amides thereof, and multimers of these. Among these, an ester of an unsaturated carboxylic acid and a polyhydric alcohol compound, amides of an unsaturated carboxylic acid and a polyvalent amine compound, and multimers of these are preferable. Furthermore, a product of an addition reaction between unsaturated carboxylic acid esters or amides having a nucleophilic substituent such as a hydroxyl group, an amino group, or a mercapto group and monofunctional or polyfunctional isocyanates or epoxies, a product of a dehydrocondensation reaction between the aforementioned unsaturated carboxylic acid esters or amides and a monofunctional or polyfunctional carboxylic acid are also suitably used. In addition, a product of an addition reaction between unsaturated carboxylic acid esters or amides having an electrophilic substituent such as an isocyanate group or an epoxy group and monofunctional or polyfunctional alcohols, amines, or thiols and a product of a substitution reaction between unsaturated carboxylic acid esters or amides having a leaving substituent such as a halogen group or a tosyloxy group and monofunctional or polyfunctional alcohols, amines, or thiols are also suitable. As another example, instead of the aforementioned unsaturated carboxylic acids, it is also possible to use a group of compounds substituted with an unsaturated phosphonic acid, a vinyl benzene derivative such as styrene, vinyl ether, allyl ether.

Specific examples of the monomer of a polyhydric alcohol compound and an unsaturated carboxylic acid include an acrylic acid ester such as ethylene glycol diacrylate, triethylene glycol diacrylate, 1,3-butanediol diacrylate, tetramethylene glycol diacrylate, propylene glycol diacrylate, neopentyl glycol diacrylate, trimethylolpropane triacrylate, trimethylolpropane tri(acryloyloxypropyl) ether, trimethylolethane triacrylate, hexanediol diacrylate, 1,4-cyclohexanediol diacrylate, tetraethylene glycol diacrylate, pentaerythritol diacrylate, pentaerythritol triacrylate, dipentaerythritol diacrylate, dipentaerythritol hexaacrylate, pentaerythritol tetraacrylate, sorbitol triacrylate, sorbitol tetraacrylate, sorbitol pentaacrylate, sorbitol hexaacrylate, tri(acryloyloxyethyl) isocyanurate, isocyanuric acid ethylene oxide (EO)-modified triacrylate, a polyester acrylate oligomer.

Specific examples of the aforementioned monomer include a methacrylic acid ester such as tetramethylene glycol dimethacrylate, triethylene glycol dimethacrylate, neopentyl glycol dimethacrylate, trimethylolpropane trimethacrylate, trimethylolethane trimethacrylate, ethylene glycol dimethacrylate, 1,3-butanediol dimethacrylate, hexanediol dimethacrylate, pentaerythritol dimethacrylate, pentaerythritol trimethacrylate, pentaerythritol tetramethacrylate, dipentaerythritol dimethacrylate, dipentaerythritol hexamethacrylate, sorbitol trimethacrylate, sorbitol tetramethacrylate, bis[p-(3-methacryloxy-2-hydroxypropoxy)phenyl]dimethylmethane, bis-[p-(methacryloxyethoxy)phenyl] dimethylmethane.

Specific examples of the aforementioned monomer include an itaconic acid ester such as ethylene glycol diitaconate, propylene glycol diitaconate, 1,3-butanediol diitaconate, 1,4-butanediol diitaconate, tetramethylene glycol diitaconate, pentaerythritol diitaconate, sorbitol tetraitaconate.

Specific examples of the aforementioned monomer include a crotonic acid ester such as ethylene glycol dicrotonate, tetramethylene glycol dicrotonate, pentaerythritol dicrotonate, sorbitol tetradicrotonate.

Specific examples of the aforementioned monomer include an isocrotonic acid ester such as ethylene glycol diisocrotonate, pentaerythritol diisocrotonate, sorbitol tetraisocrotonate.

Specific examples of the aforementioned monomer include a maleic acid ester such as ethylene glycol dimaleate, triethylene glycol dimaleate, pentaerythritol dimaleate, sorbitol tetramaleate.

As the ester of an unsaturated carboxylic acid, a monomer having a hydroxyl group can also be preferably used, and examples thereof include 2-hydroxyethyl (meth)acrylate, 2-hydroxypropyl (meth)acrylate, 4-hydroxybutyl (meth)acrylate, 6-hydroxyhexyl (meth)acrylate.

As other esters, for example, the aliphatic alcohol-based esters described in JP1971-27926B (JP-S46-27926B), JP1976-47334B (JP-S51-47334B), and JP1982-196231A (JP-S57-196231A), the esters having an aromatic skeleton described in JP1984-5240A (JP-S59-5240A), JP1984-5241A (JP-S59-5241A), and JP1990-2261449A (JP-H02-2261449A), the esters containing an amino group described in JP1989-165613A (JP-H01-165613A), are also suitably used.

As commercially available products, A-DCP, DCP, and A-DPH (all manufactured by SHIN-NAKAMURA CHEMICAL CO., LTD.) can be used.

Specific examples of the monomer of a polyvalent amine compound and an unsaturated carboxylic acid include methylenebis-acrylamide, methylenebis-methacrylamide, 1,6-hexamethylenebis-acrylamide, 1,6-hexamethylenebis-methacrylamide, diethylenetriamine trisacryalmide, xylylene bisacrylamide, xylylene bismethacrylamide, the polymerizable compounds 1 to 12 described in paragraph "0031" in the journal of technical disclosure of Japan Institute for Promoting Invention and Innovation (technical disclosure No. 2013-502654, published in August 27, 2013, Japan Institute for Promoting Invention and Innovation), the polyfunctional compounds 1 to 11 described in paragraph "0192" in the journal of technical disclosure (technical disclosure No. 2013-502654) of Japan Institute for Promoting Invention and Innovation, N-(2-acetamidoethyl)-N-(2-hydroxyethyl)acrylamide.

Examples of other preferable amide-based monomers include the monomers having a cyclohexylene structure described in JP1979-21726B (JP-S54-21726B).

Furthermore, monomers having a betaine structure such as N-methacryloyloxyethyl-N,N-dimethylammonium-N-methylcarboxybetaine, 2-(methacryloyloxy)ethyl-2-trimethylammonio)ethylphosphate, N-(3-sulfopropyl)-N-(methacryloxyethyl-N,N-dimethylammoniumbetaine, and N-(4-sulfobutyl)-N-(methacryloylaminopropyl)-N,N-diammoniumbetaine may also be used.

In addition, urethane-based addition-polymerizable compounds manufactured using an addition reaction between an isocyanate and a hydroxyl group are also suitable. Specific examples of the compounds include the vinylurethane compound described in JP1973-41708B (JP-S48-41708B), which is obtained by adding a hydroxyl group-containing vinyl monomer represented by General Formula (A) to a polyisocyanate compound having two or more isocyanate groups in one molecule and contains two or more polymerizable vinyl groups in one molecule.

CH₂=C(R⁴)COOCH₂CH(R⁵)OH (A)

In General Formula (A), R⁴ and R⁵ represent H or CH₃.

Furthermore, the urethane acrylates described in JP1976-37193A (JP-S51-37193A), JP1990-32293B (JP-H02-32293B), and JP1990-16765B (JP-H02-16765B) and the urethane compounds having an ethylene oxide-based skeleton described in JP1983-49860B (JP-S58-49860B), JP1981-17654B (JP-S56-17654B), JP1987-39417B (JP-S62-39417B), and JP1987-39418B (JP-S62-39418B) are also suitable.

As the radically polymerizable monomer, the compounds described in paragraphs "0095" to "0108" in JP2009-288705A can also be suitably used in the present invention.

As the aforementioned radically polymerizable monomer, an ethylenically unsaturated group-containing compound is also preferable which has at least one addition-polymerizable ethylene group and has a boiling point of equal to or higher than 100°C at normal pressure. Examples of such a compound include a monofunctional (meth)acrylate such as polyethylene glycol mono(meth)acrylate, polypropylene glycol mono(meth)acrylate, and phenoxyethyl (meth)acrylate; a polyfunctional (meth)acrylate such as those obtained by adding ethylene oxide or propylene oxide to a polyfunctional alcohol such as polyethylene glycol di(meth)acrylate, trimethylolethane tri(meth)acrylate, neopentyl glycol di(meth)acrylate, pentaerythritol tri(meth)acrylate, pentaerythritol tetra(meth)acrylate, dipentaerythritol penta(meth)acrylate, dipentaerythritol hexa(meth)acrylate, hexanediol (meth)acrylate, trimethylolpropane tri(acryloyloxypropyl)ether, tri(acryloyloxyethyl)isocyanurate, glycerin, or trimethylolethane and then (meth)acrylating the resulting product, the urethane (meth)acrylates described in JP1973-41708B (JP-S48-41708B), JP1975-6034B (JP-S50-6034B), and JP1976-37193A (JP-S51-37193A), the polyester acrylates described in JP1973-64183A (JP-S48-64183A), JP1974-43191B (JP-S49-43191B), and JP1977-30490B (JP-S52-30490B), and epoxyacrylates which are products of a reaction between an epoxy resin and a (meth)acrylic acid; and a mixture of these.

Examples of the radically polymerizable monomer also include a polyfunctional (meth)acrylate obtained by reacting a polyfunctional carboxylic acid with a cyclic ether group such as glycidyl (meth)acrylate and a compound having an ethylenically unsaturated group.

Furthermore, as other preferable radically polymerizable monomers, it is possible to use a compound having a fluorene ring and two or more ethylenic polymerizable groups as functional groups that is described in JP2010-160418A, JP2010-129825A, and JP4364216B and a Cardo resin.

In addition, examples of the radically polymerizable monomer also include the specific unsaturated compounds described in JP1971-43946B (JP-S46-43946B), JP1989-40337B (JP-H01-40337B), and JP1989-40336B (JP-H01-40336B), the vinyl phosphonic acid-based compound described in JP1990-25493A (JP-H02-25493A), and the like. In some cases, the perfluoroalkyl group-containing structure described in JP1986-22048A (JP-S61-22048A) is suitably used. Moreover, it is possible to use those introduced as photocurable monomers and oligomers in the Journal of The Adhesion Society of Japan, vol. 20, No. 7, pp. 300∼308 (1984).

As the compound having a boiling point of equal to or higher than 100°C at normal pressure and having at least one addition-polymerizable ethylenically unsaturated group, the compounds described in paragraphs "0254" to "0257" in JP2008-292970A are also suitable.

Furthermore, radically polymerizable monomers represented by General Formulae (MO-1) to (MO-5) can be suitably used. In the following formulae, in a case where T is an oxyalkylene group, the terminal on the carbon atom side is bonded to R.

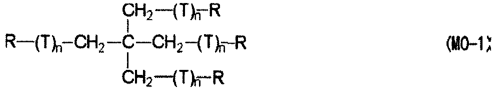

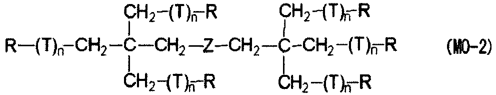

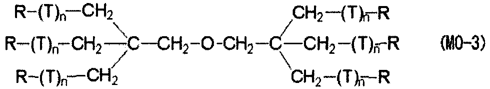

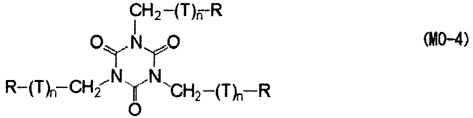

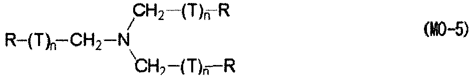

R: 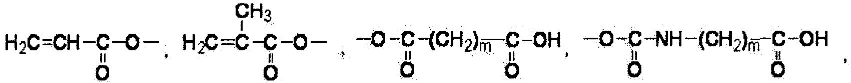 -OH -CH₃
T: 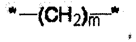 -OCH₂- , -OCH₂CH₂-, -OCH₂CH₂CH₂-, -OCH₂CH₂CH₂CH₂-, 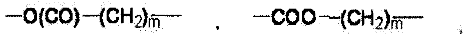 -OCH(CH₃)-CH₂-, -OCH₂CH(CH₃)-
Z: **-**O**-**,

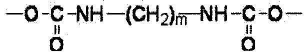

In the above general formulae, n is 0 to 14, and m is 1 to 8. A plurality of R's and T's in one molecule may be the same as or different from each other.

In each of the radically polymerizable monomers represented by General Formulae (MO-1) to (MO-5), at least one of the plurality of R's represents a group represented by -OC(=O)CH=CH₂ or -OC(=O)C(CH₃)=CH₂.

Specific examples of the radically polymerizable monomers represented by General Formula (MO-1) to (MO-5) include the compounds described in paragraphs "0248" to "0251" in JP2007-269779A, and these compounds can also be suitably used in the present invention.

In addition, the compounds, which are described as General Formulae (1) and (2) in JP1998-62986A (JP-H10-62986A) together with the specific examples thereof and obtained by adding ethylene oxide or propylene oxide to the aforementioned polyfunctional alcohol and then (meth)acrylating the resulting product, can also be used as the radically polymerizable monomer.

Among these, as the radically polymerizable monomer, dipentaerythritol triacrylate (KAYARAD D-330 as a commercially available product; manufactured by Nippon Kayaku Co., Ltd.), dipentaerythritol tetraacrylate (KAYARAD D-320 as a commercially available product; manufactured by Nippon Kayaku Co., Ltd.), dipentaerythritol penta(meth)acrylate (KAYARAD D-310 as a commercially available product; manufactured by Nippon Kayaku Co., Ltd.), dipentaerythritol hexa(meth)acrylate (KAYARAD DPHA as a commercially available product; manufactured by Nippon Kayaku Co., Ltd.), and a structure in which a (meth)acryloyl group of these is bonded through an ethylene glycol residue or a propylene glycol residue are preferable. The oligomer type of these can also be used.

The radically polymerizable monomer may be a polyfunctional monomer having a residue such as a carboxyl group, a sulfonic acid group, and a phosphoric acid group. Accordingly, as long as an ethylenic compound has an unreacted carboxyl group as in a case where the compound is a mixture described above, the ethylenic compound can be used as is. However, if necessary, a residue may be introduced into the compound by reacting a non-aromatic carboxylic acid anhydride with a hydroxyl group of the aforementioned ethylenic compound. In this case, specific examples of the non-aromatic carboxylic acid anhydride used include tetrahydrophthalic acid anhydride, alkylated tetrahydrophthalic acid anhydride, hexahydrophthalic acid anhydride, alkylated hexahydrophthalic acid anhydride, succinic acid anhydride, and maleic acid anhydride.

In the present invention, the monomer may have a charge by containing either a group having an acid value or a cationic group. Examples of the monomer having an acid value include an ester of an aliphatic polyhydroxy compound and an unsaturated carboxylic acid. As the monomer, a polyfunctional monomer is preferable which is obtained by reacting an unreacted hydroxyl group of an aliphatic polyhydroxy compound with a non-aromatic carboxylic acid anhydride such that an acid group is introduced into the monomer, and the aforementioned ester is particularly preferable in which the aliphatic polyhydroxy compound is pentaerythritol and/or dipentaerythritol. Examples of commercially available products thereof include M-510 and M-520 which are polybasic acid-modified acryl oligomers manufactured by TOAGOSEI CO., LTD.

One kind of these monomers may be used singly. However, for the sake of manufacturing, it is difficult to use a single compound. Therefore, two or more kinds of these monomers may be used by being mixed together. Furthermore, if necessary, as the monomer, a polyfunctional monomer not having an acid group and a polyfunctional monomer having an acid group may be used in combination.

The acid value of the polyfunctional monomer having an acid group is preferably 0.01 to 40 mg-KOH/g, and particularly preferably 0.1 to 30 mg-KOH/g. In a case where the acid value of the polyfunctional monomer is too low, the dispersibility of the monomer in water deteriorates. In a case where the acid value of the polyfunctional monomer is too high, it is difficult to manufacture or handle the monomer, the photopolymerization performance deteriorates, and the curing properties such as surface smoothness becomes poor. Accordingly, in a case where two or more kinds of polyfunctional monomers having different acid groups are used in combination or in a case where polyfunctional monomers not having an acid group are used in combination, the acid value of all the polyfunctional monomers can be adjusted and fall into the aforementioned range.

Furthermore, it is preferable that the curable composition contains a polyfunctional monomer having a caprolactone structure as the radically polymerizable monomer.

The polyfunctional monomer having a caprolactone structure is not particularly limited as long as the monomer has a caprolactone structure in the molecule thereof. Examples thereof include an ε-caprolactone-modified polyfunctional (meth)acrylate obtained by esterifying a polyhydric alcohol such as trimethylolethane, ditrimethylolethane, trimethylolpropane, ditrimethylolpropane, pentaerythritol, dipentaerythritol, tripentaerythritol, glycerin, diglycerol, and trimethylol melamine, (meth)acrylic acid, and ε-caprolactone. Among these, a polyfunctional monomer having a caprolactone structure represented by Formula (1) is preferable.

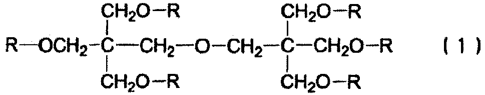

In General Formula (1), all of six R's represent a group represented by General Formula (2). Alternatively, one to five among six R's represent a group represented by General Formula (2), and the rest represent a group represented by General Formula (3).

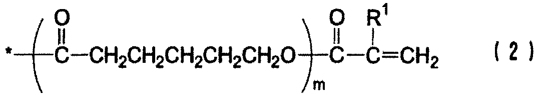

In General Formula (2), R¹ represents a hydrogen atom or a methyl group, m represents number 1 or 2, and "*" represents a bond.

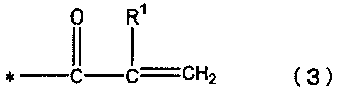

In General Formula (3), R¹ represents a hydrogen atom or a methyl group, and "*" represents a bond.

The polyfunctional monomer having a caprolactone structure is available on the market as a KAYARAD DPCA series from Nippon Kayaku Co., Ltd., for example. Examples thereof include DPCA-20 (a compound in which m in Formulae (1) to (3) = 1, the number of groups represented by Formula (2) = 2, and all the R¹'s represent a hydrogen atom), DPCA-30 (a compound in which m in Formulae (1) to (3) = 1, the number of groups represented by Formula (2) = 3, and all the R¹'s represent a hydrogen atom), DPCA-60 (a compound in which m in Formulae (1) to (3) = 1, the number of groups represented by Formula (2) = 6, and all the R¹'s represent a hydrogen atom), DPCA-120 (a compound in which m in Formulae (1) to (3) = 2, the number of groups represented by Formula (2) = 6, and all the R¹'s represent a hydrogen atom).

In the present invention, one kind of polyfunctional monomer having a caprolactone structure can be used singly, or two or more kinds thereof may be used by being mixed together.

In addition, the polyfunctional monomer is preferably at least one kind of compound selected from the group consisting of compounds represented by General Formulae (i) or (ii).

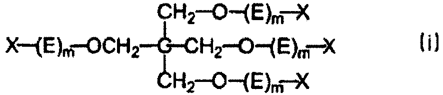

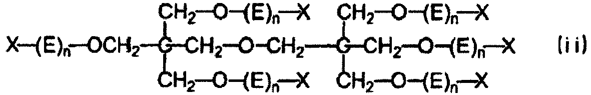

In General Formulae (i) and (ii), E each independently represents -((CH₂)_{y}CH₂O)- or -((CH₂)_{y}CH(CH₃)O)-, y each independently represents an integer of 0 to 10, and X each independently represents an acryloyl group, a methacryloyl group, a hydrogen atom, or a carboxyl group.

In General Formula (i), the total number of acryloyl groups and methacryloyl groups is 3 or 4, m each independently represents an integer of 0 to 10, and the total number of m's is an integer of 0 to 40. Here, in a case where the total number of m's is 0, any one of X's is a carboxyl group.

In General Formula (ii), the total number of acryloyl groups and methacryloyl groups is 5 or 6, n each independently represents an integer of 0 to 10, and the total number of n's is an integer of 0 to 60. Here, in a case where the total number of n's is 0, any one of X's is a carboxyl group.

In General Formula (i), m is preferably an integer of 0 to 6, and more preferably an integer of 0 to 4. The total number of m's is preferably an integer of 2 to 40, more preferably an integer of 2 to 16, and particularly preferably an integer of 4 to 8.

In General Formula (ii), n is preferably an integer of 0 to 6, and more preferably an integer of 0 to 4. The total number of n's is preferably an integer of 3 to 60, more preferably an integer of 3 to 24, and particularly preferably an integer of 6 to 12.

-((CH₂)_{y}CH₂O)- or -((CH₂)_{y}CH(CH₃)O)- in General Formula (i) or (ii) is preferably in the form in which the terminal thereof on the oxygen atom side is bonded to X.

One kind of compound represented by General Formula (i) or (ii) may be used singly, or two or more kinds thereof may be used in combination. Particularly, an aspect is preferable in which all of six X's in General Formula (ii) represent an acryloyl group.

The total content of the compound represented by General Formula (i) or (ii) in the radically polymerizable monomer is preferably equal to or greater than 20% by mass, and more preferably equal to or greater than 50% by mass.

The compound represented by General Formula (i) or (ii) can be synthesized through the steps known in the related art, including a step of bonding a ring-opening skeleton to pentaerythritol or dipentaerythritol by causing a ring-opening addition reaction between the pentaerythritol or the dipentaerythritol and ethylene oxide or propylene oxide and a step of introducing a (meth)acryloyl group into the resulting product by reacting a terminal hydroxyl group of the ring-opening skeleton with, for example, (meth)acryloyl chloride. Each of these steps is well known, and those skilled in the art can easily synthesize the compound represented by General Formula (i) or (ii).

Among the compounds represented by General Formula (i) or (ii), either or both of a pentaerythritol derivative and dipentaerythritol derivative are more preferable.

Specifically, examples thereof include compounds represented by General Formulae (a) to (f) (hereinafter, referred to as "example compounds (a) to (f)" as well), and among these, the example compound (a), (b), (e), or (f) is preferable.

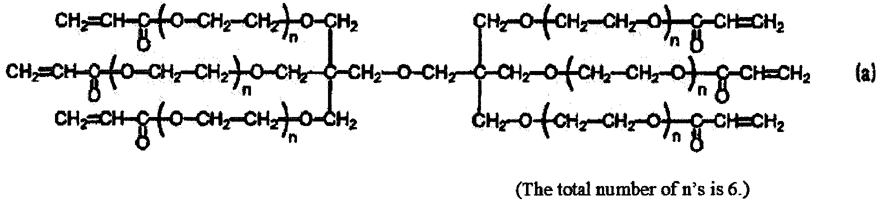

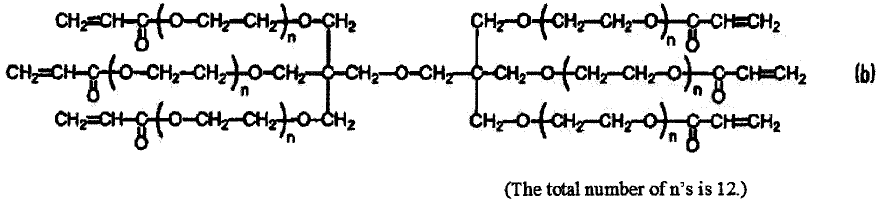

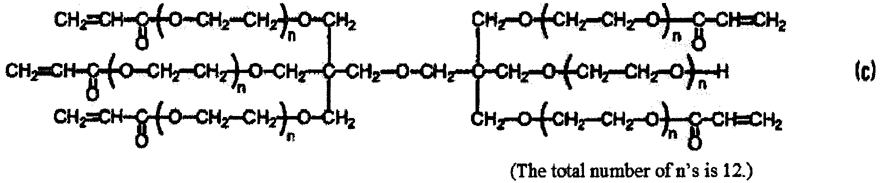

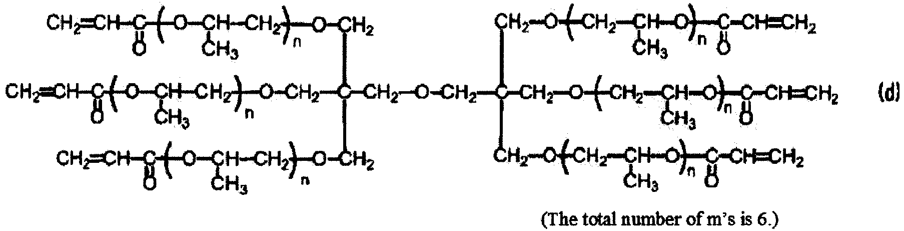

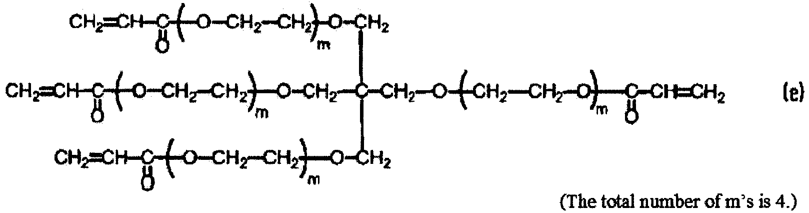

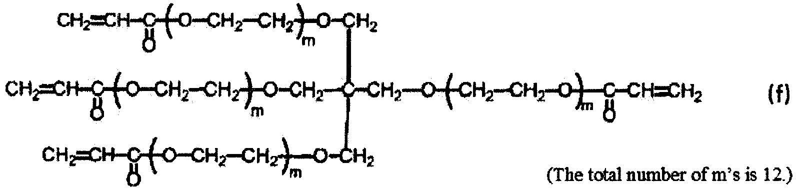

Examples of commercially available products of the radically polymerizable monomer represented by General Formula (i) or (ii) include SR-494 manufactured by Sartomer that is a tetrafunctional acrylate having four ethyleneoxy chains, DPCA-60 manufactured by Nippon Kayaku Co., Ltd. that is a hexafunctional acrylate having six pentyleneoxy chains, TPA-330 manufactured by Nippon Kayaku Co., Ltd. that is a trifunctional acrylate having three isobutyleneoxy chains.

As the polyfunctional monomer, a polyfunctional (meth)acrylamide is also preferably used. The polyfunctional (meth)acrylamide is not particularly limited as long as it has two or more (preferably two to six) (meth)acrylamide groups. In the present specification, conceptually, the (meth)acrylamide includes both the acrylamide and the methacrylamide.

Among polyfunctional (meth)acrylamides, a tetrafunctional (meth)acrylamide represented by General Formula (E) can be more preferably used because this monomer improves the curing speed of the curable composition.

The tetrafunctional (meth)acrylamide represented by General Formula (E) can be manufactured by the manufacturing method described in JP5486536B, for example.

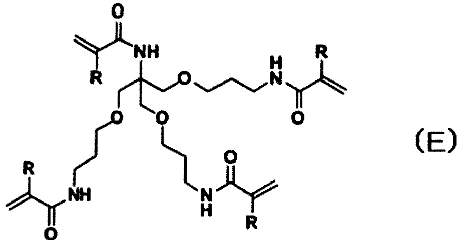

In General Formula (E), R represents a hydrogen atom or a methyl group. In General Formula (E), a plurality of R's may be the same as or different from each other.

The tetrafunctional (meth)acrylamide represented by General Formula (E) has excellent water solubility and is highly compatible with the specific polymerization initiator and the specific additive of the present invention. Presumably, for this reason, in a case where an aqueous solvent is used as the active energy ray-curable composition, the curing speed of a cured product (cured film) obtained using the tetrafunctional (meth)acrylamide is increased.

As the radically polymerizable monomer, the urethane acrylates described in JP1973-41708B (JP-S48-41708B), JP1976-37193A (JP-S51-37193A), JP1990-32293B (JP-H02-32293B), and JP1990-16765B (JP-H02-16765B) and the urethane compounds having an ethylene oxide-based skeleton described in JP1983-49860B (JP-S58-49860B), JP1981-17654B (JP-S56-17654B), JP1987-39417B (JP-S62-39417B), and JP1987-39418B (JP-S62-39418B) are also suitable. Furthermore, as the polymerizable compound, it is also possible to use the addition-polymerizable compounds having an amino structure or a sulfide structure in a molecule described in JP1988-277653A (JP-S63-277653A), JP1988-260909A (JP-S63-260909A), and JP1989-105238A (JP-H01-105238A).

Examples of commercially available products of the radically polymerizable monomer include urethane oligomers UAS-10 and UAB-140 (manufactured by Sanyo Kokusaku Pulp Co., Ltd.), UA-7200 (manufactured by SHIN-NAKAMURA CHEMICAL CO., LTD.), DPHA-40H (manufactured by Nippon Kayaku Co., Ltd.), UA-306H, UA-306T, UA-306I, AH-600, T-600, and AI-600 (manufactured by KYOEISHA CHEMICAL Co., LTD.

Regarding the radically polymerizable monomer, the details of how the monomer is used, such as the structure of the monomer, whether one kind of the monomer is used singly or plural kinds of the monomers are used in combination, and the amount of the monomer added, can be arbitrarily set according to the final performance design for the pressure sensitive adhesive composition. For example, from the viewpoint of sensitivity (efficiency of the decrease in sensitivity with respect to the irradiation of active energy rays or radiation), a structure is preferable in which a large amount of unsaturated groups are contained in one molecule, and in many cases, a radically polymerizable monomer having two or more functional groups is preferable. Furthermore, from the viewpoint of improving the hardness of a pressure sensitive adhesive layer, a radically polymerizable monomer having three or more functional groups is preferable. In addition, it is effective to use a method of regulating both the sensitivity and the hardness by using radically polymerizable monomers having a different number of functional groups and/or different polymerizable groups (for example, an acrylic acid ester, a methacrylic acid ester, a styrene-based compound, and a vinyl ether-based compound) in combination. Moreover, it is preferable to use radically polymerizable monomers having three or more functional groups and having ethylene oxide chains of different lengths. For the compatibility with other components (for example, a polymer, a polymerization initiator, and the like) contained in the pressure sensitive adhesive composition and the dispersibility, the selection of the radically polymerizable monomer and how the monomer is used are important factors. For example, in some cases, by using a compound with low purity or using two or more kinds of radically polymerizable monomers, the compatibility can be further improved. Furthermore, from the viewpoint of improving the adhesiveness with respect to a support, a specific structure can be selected.

The content of the polymerizable compound may be appropriately determined according to the use of the curable composition. For example, the content of the polymerizable compound with respect to the mass (100% by mass) of the total solid contents in the curable composition is preferably 10% to 99.9% by mass, more preferably 15 to 99.8% by mass, and even more preferably 20% to 99.7% by mass.

In the present specification, the solid contents mean the components constituting a cured product (cured film) formed of the curable composition and do not include a solvent. For example, being a component constituting the cured product (cured film), the polymerizable compound is included in the solid contents even though it is a liquid (in a liquid form).

### <Other components>

If necessary, the curable composition of the present invention may contain components other than the aforementioned components, for example, a solvent (water, an organic solvent), a surfactant (a fluorine-based surfactant, a nonionic surfactant, a cationic surfactant, an anionic surfactant, a silicone-based surfactant), a sensitizing dye (merocyanines, benzopyrans, coumarins, aromatic ketones, anthracenes, styryls, oxazoles, and the like), a chain transfer agent (a thiol-based compound such as 2-mercaptobenzimidazoles, 2-mercaptobenzothiazoles, 2-mercaptobenzoxazoles, 3-mercaptotriazoles, and 5-mercaptotetrazoles), a polymerization inhibitor (hydroquinone, p-methoxyphenol, di-t-butyl-p-cresol, pyrogallol, t-butylcatechol, benzoquinone, 4,4'-thiobis(3-methyl-6-tert-butylphenol), 2,2'-methylenebis(4-methyl-6-tert-butylphenol), a N-nitroso-N-phenylhydroxyamine aluminum salt) a higher fatty acid derivative (behenic acid, behenic acid amide), and a cross-linking agent other than a specific cross-linking agent (a carbodiimide-based cross-linking agent, an oxazoline-based cross-linking agent, an aziridine-based cross-linking agent, a melamine resin-based cross-linking agent, a metal chelate-based cross-linking agent, a peroxide-based cross-linking agent).

Furthermore, the curable composition of the present invention may contain various components such as a binder, a curing agent, a curing catalyst, a silane coupling agent, a filler, an adhesion accelerator, an antioxidant, an ultraviolet absorber, an aggregation inhibitor, a viscosity imparting agent, an anti-aging agent, a colorant (a pigment, a dye) a release controlling agent, a plasticizer, and a softener.

### <Preparation method>

The curable composition of the present invention is obtained by mixing the aforementioned components together and stirring the mixture, and the preparation method thereof is not particularly limited.

### [Use]

Next, the use of the photopolymerization initiator composition and the curable composition of the present invention will be described.

The photopolymerization initiator composition of the present invention is preferably used in the curable composition which will be described later. In this case, the photopolymerization initiator composition may be used in a solventless curable composition not containing a solvent (water, an organic solvent, and the like), in an aqueous curable composition mainly composed of water (the content of water in all solvents is greater than 50% by mass), or in an organic solvent-based curable composition mainly composed of an organic solvent (the content of an organic solvent in all solvents is greater than 50% by mass).

The use of the curable composition of the present invention is not particularly limited, and examples thereof include paints, adhesives, pressure sensitive adhesives, inks, coatings, functional membranes, films, optical materials, printing plate materials, semiconductor materials, recording materials, paper additives, medical materials, plastics, functional gels, and the like.

Examples of the uses of the photopolymerization initiator composition and the curable composition of the present invention will be listed below, but the uses of the photopolymerization initiator composition and the curable composition of the present invention is not limited thereto.

Examples of the paints include the self-repairable paint described in JP2013-049839A, JP2011-005766A, the wood working paint described in JP2006-007202A, JP5072140B, the antibacterial paint described in JP2011-057855A the stream-resistance inhibitory paint described in JP1999-019578A (JP-H11-019578A).

Examples of the adhesives include the dental adhesive described in JP2010-235458A, JP4664591B, JP2013-056839A, JP4171600B, the medical adhesive described in JP2011-026551A, JP2009-247437A, the adhesive for recording materials described in JP2011-198434A, JP2011-165238A, the adhesive for optical members described in JP5491525B and JP2012-46658A.

Examples of the pressure sensitive adhesives include the pressure sensitive adhesive for hardcoat films described in JP2013-032500A, JP2013-040256A, the pressure sensitive adhesive for dicing tapes described in JP2011-089073A the pressure sensitive adhesive composition for optical members described in WO2010/092995A and WO2010/092988A.

Examples of the inks include the ink jet ink described in JP2002-241654A, JP2010-106085A, the printing ink described in JP1998-17605A (JP-H10-17605A), JP2002-285062A, the overprint varnish described in JP1998-195371A(JP-H10-195371A).

Examples of the coatings include the coating agent for optical fiber described in JP2012-136426A, JP2006-208663A, the buffer coating agent described in JP2011-116968A the glazing described in JP2012-229331A, JP2011-074135A, the coating for headlights described in JP2011-241356A, JP2002-212507A, the coating for building materials described in JP2011-088995A, JP2011-213002A, the hardcoating agent for cosmetic containers described in JP2008-303310A, JP2006-1984A, the coating agent for electronic instruments described in JP2011-225846A the overcoating agent for silver-based transparent conductive films described in JP2013-65305A, JP2013-22843A, the sealing material for electronic instruments described in JP2012-000828A, JP2010-278157A, the coating agent for kitchen appliances described in JP2011-094125A, JP2004-211025A, the hybrid hardcoating agent described in JP1999-194491A (JP-H11-194491A), the antifouling coating described in JP2010-095707A, JP2012-219116A, the coating material with high weather fastness described in JP2013-035267A, JP2012-167288A, the antistatic agent described in JP4600606B and JP2010-229187A.

Examples of the functional membranes include the reverse osmosis membrane described in JP4070009B, JP2014-069155A, the proton conductive membrane described in JP5346194B, JP4014422B, the porous membrane described in JP5014576B.

Examples of the films include the heat shielding film described in JP2012-128231A, JP1998-100310A (JP-H10-100310A), the hardcoat film described in JP2013-075955A, JP2012-197383A, JP2013-06821A, JP2013-050641A, the antireflection film described in JP2013-083795A, JP2013-033098A, the diffusion film described in JP2012-098526A, JP2012-078799A, the peeling film described in JP2012-250353A, JP2011-178002A, the solar cell back sheet described in JP2012-227382A the electromagnetic wave shield described in JP2011-124536A, JP2010-147431A, the gas barrier film described in JP5116410B, the film for packaging foods and cosmetics described in JP2008-150610A the antifogging film described in JP2000-154373A the optical film described in JP2008-165205A and JP2012-150428A.

Examples of the optical materials include the optical lens described in JP2006-233172A.

Examples of the printing plate materials include the image forming material described in JP1998-221850A (JP-H10-221850A), JP5381362B, JP2009-226946A,

Examples of the semiconductor materials include the dry film resist described in JP2009-048170A, the resist underlayer film described in JP2013-083947A, JP2012-203393A, the nanoimprinting material described in JP2013-062489A, JP2012-214022A, the color resist material described in JP2012-027448A, JP2010-204363A, JP2013-053224A, JP2011-095732A, the solder resist material described in JP2009-217040A.

Examples of the recording materials include the hardcoating agent for recording media described in JP2011-192342A, JP2011-126991A, JP1994-128501A (JP-H06-128501 A).

Examples of the paper additives include the recording paper coating agent described in JP1999-115305A (JP-H11-115305A), JP3647125B.

Examples of the medical materials include the formulation described in JP2012-011269A the artificial bone described in JP2008-535979A, JP5502768B, the contact lens described in JP4988025B, the curable composition for embedding described in JP2000-346770.

Examples of the plastics include the composition for three-dimensional modeling described in JP2010-155889A.

Examples of the functional gels include the electrolyte gel described in JP2008-285668A the hygroscopic gel described in JP1997-077832A (JP-H09-077832A), the ionic gel described in JP2001-000406A, JP1997-140681A (JP-H09-140681A), the woodworking gel described in JP1995-242873A (JP-H07-242873A), the medical gel described in JP2011-197196A.

For the use of lens, for example, by combining the initiator described in JP2011-072341 with the photopolymerization initiator composition of the present invention, lenses can be prepared. This shows that the photopolymerization initiator composition of the present invention is also useful as the medical materials described in JP2011-072341A (an ocular lens, an endoscope, a catheter, an infusion tube, a gas transportation tube, a stent, a sheath, a cuff, a tube connector, an access port, a drainage bag, a blood circuit, a wound dressing, and a medicine carrier).

The photopolymerization initiator composition of the present invention has high hydrophilicity. Therefore, the composition is useful in uses for which an uncured portion needs to be washed off with water. For example, by combining the initiator described in JP2011-197196A with the photopolymerization initiator composition of the present invention, it is possible to obtain a preferable medical material such as a contact lens. Furthermore, by combining the initiator described in JP2012-111226A with the photopolymerization initiator composition of the present invention, it is possible to obtain a preferable water-soluble composition for three-dimensional modeling.

The photopolymerization initiator composition of the present invention excellently permeates a substrate. Therefore, for example, by combining the initiator described in JP2012-066235A with the photopolymerization initiator composition of the present invention, it is possible to obtain a preferable paint for automobiles.

Furthermore, the photopolymerization initiator composition of the present invention can be used as is or can be usefully applied in uses after modifying the OH group on the terminal thereof. For example, by combining the initiator described in JP2001-163932A with the photopolymerization initiator composition of the present invention in which the OH groups of the terminals of the respective components contained in the composition are modified, it is possible to obtain a preferable coating for contact lenses. In addition, by combining the initiator described in JP5089710B with the photopolymerization initiator composition of the present invention, it is possible to obtain a preferable pressure sensitive adhesive for dicing tapes.

### [Cured product]

The cured product of the present invention can be obtained by curing the aforementioned curable composition (active energy ray-curable composition). On the cured product of the present invention, bleed out occurs less after the thermocycling test. Therefore, even in a case where the cured product is applied in various uses described above, it is possible to inhibit the occurrence of problems resulting from the cured product. Accordingly, the cured product of the present invention is suitable for the various uses described above.

The method for manufacturing the cured product of the present invention preferably includes a step of irradiating the curable composition with active energy rays. In the manufacturing method, a drying treatment or a heating treatment may be performed as appropriate.

Various conditions (irradiation conditions, drying conditions, heating conditions, and the like) at the time of manufacturing the cured product of the present invention are not particularly limited, and may be appropriately set according to the type and content ratio of the components contained in the curable composition, the use of the cured product.

### Examples

Hereinafter, the photopolymerization initiator composition of the present invention will be specifically described using examples, but the present invention is not limited thereto.

### [Synthesis of photopolymerization initiator]

### <Synthesis of photopolymerization initiator (I-1) (Synthesis Example 1)>

By the synthesis method described below, a photopolymerization initiator composition (I-1) as a type of photopolymerization initiator composition represented by General Formula (I) was obtained.

First, 1.1 moles of a triethylamine solution dissolved in 500 ml of tetrahydrofuran was added dropwise to a mixture of 1 moles of Irgacure 2959 (trade name, manufactured by BASF SE) and 0.66 moles of methansulfonic acid chloride at room temperature, thereby obtaining a reaction mixture.

The reaction mixture was stirred for 3 hours, and diluted with 500 ml of water and then with 300 ml of methyl ethyl ketone. By evaporating the solvents, a crude product was obtained, and the crude product was recrystallized from isopropanol, thereby obtaining 4-(2-methylsulfonyloxyethoxy)-phenyl-2-hydroxy-2-propylketone. By adding 90 ml of ethylene glycol containing 2.4 g of sodium (0.099 atomic equivalent) thereto, a suspension was obtained.

The obtained suspension was heated for 2 hours at 40°C, and 0.09 moles of 4-(2-methylsulfonyloxyethoxy)-phenyl-2-hydroxy-2-propylketone was added to the solution. The mixture was heated for 2 hours at a temperature of 70°C to 80°C, then diluted with 50 ml of water, and subjected to extraction using diethyl ether. The solvents were evaporated, and then the obtained crude product was purified by flash chromatography (silica gel, eluent: ethyl acetate), thereby obtaining the photopolymerization initiator (I-1) (Formula (I-1)) as an oily substance.

### <Polymerization initiator other than polymerization initiator (I-1)>

As the photopolymerization initiator represented by General Formula (I), photopolymerization initiators represented by Formulae (I-2) to (I-4) were synthesized by the same method as in Synthesis Example 1.

Furthermore, as photopolymerization initiators compared with the photopolymerization initiator represented by General Formula (I), a photopolymerization initiator C-1 (Formula C-1) and a photopolymerization initiator C-2 (Formula C-2) were prepared. As the photopolymerization initiator C-1, a commercially available product "IRGACURE 2959" (trade name, manufactured by BASF SE) was used. The photopolymerization initiator C-2 was synthesized by the same method as in Synthesis Example 1.

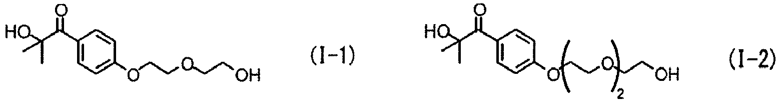

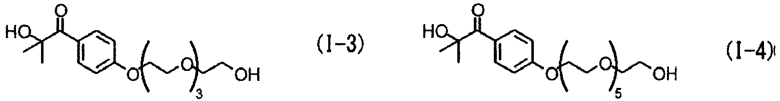

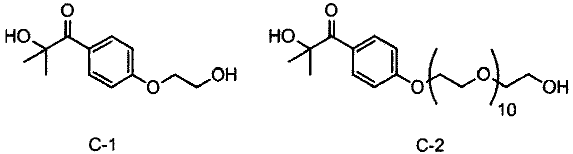

### [Synthesis of specific additive]

At room temperature, 134.15 g (0.5 moles) of the photopolymerization initiator (1-1) was dissolved in 170 ml of a 20% by mass methanol (MeOH) solution, and while the obtained solution was being stirred, 33 ml of a 30% by mass aqueous sodium hydroxide (NaOH) solution was added dropwise thereto. Then, the internal temperature was increased to 80°C, and the reaction was performed for three days, thereby obtaining a reaction solution.

Thereafter, the reaction solution was cooled to room temperature, neutralized using an aqueous hydrochloric acid solution, and subjected to extraction using ethyl acetate. The obtained organic layer was concentrated and filtered by being passed through a filter (pore size: 3 µm, material: polytetrafluoroethylene (PTFE)) manufactured by ADVANTEC MFS Inc. After the solvents were removed, the resulting solid was dissolved in tetrahydrofuran (THF) and purified by recycling preparative high-performance liquid chromatography (LC-9210 NEXT manufactured by Japan Analytical Industry Co., Ltd., packed columns: JAIGEL H1 and H2), thereby purifying a specific additive (IV-1) (Formula (IV-1)) as an example of the compound represented by General Formula (IV).

By the same synthesis method as described above, a specific additive (II-1) (Formula (II-1) as an example of the compound represented by General Formula (II) and a specific additive (III-1) (Formula (III-1)) as an example of the compound represented by General Formula (III) were obtained.

A specific additive (IV-2) (Formula (IV-2)) as an example of the compound represented by General Formula (IV) was obtained by the same synthesis method as that used for synthesizing the specific additive (IV-1), except that the polymerization initiator (1-2) was used.

A specific additive (IV-3) (Formula (IV-3)) as an example of the compound represented by General Formula (IV) was obtained by the same synthesis method as that used for synthesizing the specific additive (IV-1), except that the polymerization initiator (1-3) was used.

A specific additive (IV-4) (Formula (IV-4)) as an example of the compound represented by General Formula (IV) was obtained by the same synthesis method as that used for synthesizing the specific additive (IV-1), except that the polymerization initiator (I-4) was used.

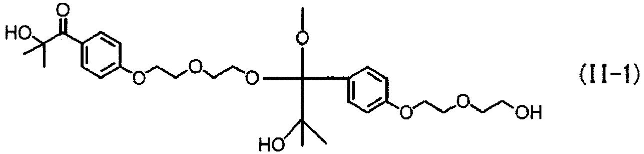

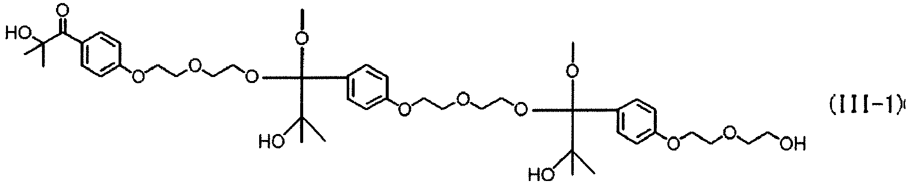

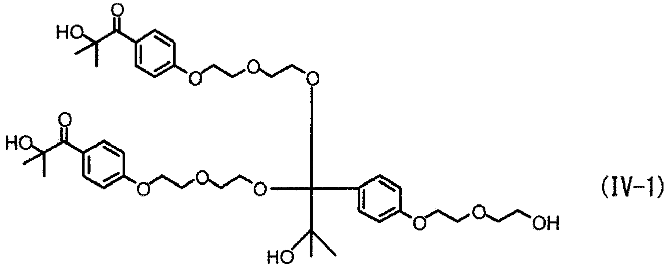

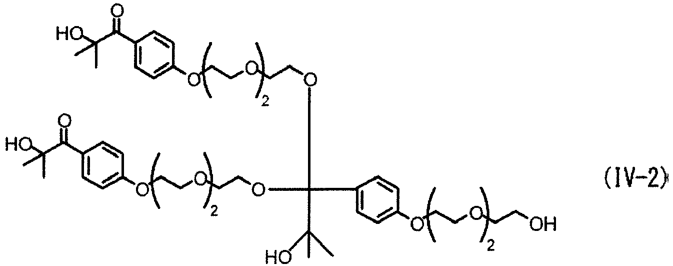

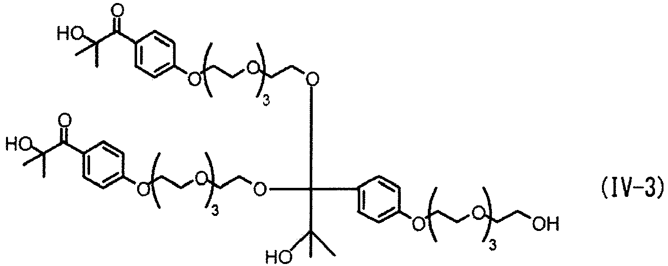

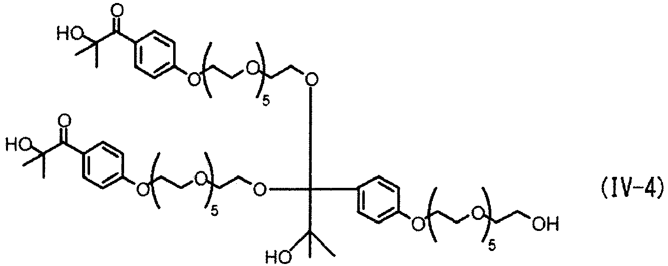

### [Preparation of photopolymerization initiator composition]

The photopolymerization initiators and the specific additives obtained as above were formulated together at the ratio shown in the first table and then mixed and stirred together, thereby obtaining photopolymerization initiator compositions (example compositions and comparative compositions).

**[Table 2]**

| First table | Photopolymerization initiator (part by mass) | | | | | | Specific additive (part by mass) | | | | | |
|---|---|---|---|---|---|---|---|---|---|---|---|---|
| Photopolymerization initiator composition | I-1 | I-2 | I-3 | I-4 | C-1 | C-2 | IV-1 | IV-2 | IV-3 | IV-4 | III-1 | II-1 |
| | n = 1 in General Formula (I) | n = 2 in General Formula (I) | n = 3 in General Formula (I) | n = 5 in General Formula (I) | n = 0 in General Formula (I) | n = 10 in General Formula (I) | (Trimer) | (Trimer) | (Trimer) | (Trimer) | (Trimer) | (Dimer) |
| Example composition 1 | 100 | | | | | | 0.02 | | | | | |
| Example composition 2 | 100 | | | | | | | | | | | 0.02 |
| Example composition 3 | 100 | | | | | | 10 | | | | | |
| Example composition 4 | 100 | | | | | | 8 | | | | | |
| Example composition 5 | 100 | | | | | | 0.001 | | | | | |
| Example composition 6 | 100 | | | | | | 0.0005 | | | | | |
| Example composition 7 | | 100 | | | | | | 0.02 | | | | |
| Example composition 8 | | | 100 | | | | | | 0.02 | | | |
| Example composition 9 | | | | 100 | | | | | | 0.02 | | |
| Example composition 10 | 100 | | | | | | | | | | 0.02 | |
| Example composition 11 | 100 | | | | | | 0.01 | | | | 0.01 | |
| Example composition 12 | 100 | | | | | | 0.01 | | | | | 0.01 |
| Example composition 13 | 100 | | | | | | 0.005 | | | | 0.005 | 0.01 |
| Comparative composition 1 | 100 | | | | | | | | | | | |
| Comparative composition 2 | | | | | 100 | | 0.02 | | | | | |
| Comparative composition 3 | | | | | | 100 | 0.02 | | | | | |

### [Preparation of active energy ray-curable composition]

According to the composition described in the second table, the aforementioned photopolymerization initiator compositions, polymerizable compounds, and solvents (water and methanol) were mixed together, thereby obtaining active energy ray-curable compositions of examples and comparative examples.

As the polymerizable compounds, 2-hydroxyethyl methacrylate (HEMA, manufactured by Wako Pure Chemical Industries, Ltd.) and an acrylamide monomer (acrylamide monomer (E-1)) represented by Formula (E-1) were used. The acrylamide monomer represented by Formula (E-1) was manufactured according to the manufacturing method described in JP5486536B.

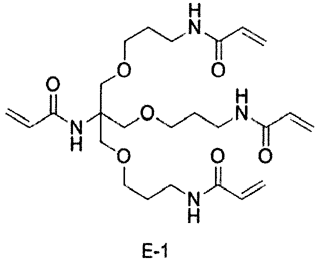

**[Table 3]**

| Second table | Active energy ray-curable composition | | | | | Evaluation result | | | |
|---|---|---|---|---|---|---|---|---|---|
| | Photopolymerization initiator composition | | Polymerizable compound | | Solvent | Bleed out after thermocycling test | Storage stability (-20°C) | Solubility in water (turbidity) | Sensitivity |
| | Type | Content (part by mass) | HEMA (part by mass) | Acrylamide monomer E-1 (part by mass) | Water/MeOH = 50/50 (part by mass) | (Measurement subject) Cured film of active energy ray-curable composition | (Measurement subject) Photopolymerization initiator composition | (Measurement subject) Photopolymerization initiator composition | (Measurement subject) Active energy ray-curable composition |
| Example 1 | Example composition 1 | 5 | 20 | 80 | 150 | 5 | A | A | A |
| Example 2 | Example composition 2 | 5 | 20 | 80 | 150 | 4 | A | A | B |
| Example 3 | Example composition 3 | 5 | 20 | 80 | 150 | 5 | A | B | B |
| Example 4 | Example composition 4 | 5 | 20 | 80 | 150 | 5 | A | B | A |
| Example 5 | Example composition 5 | 5 | 20 | 80 | 150 | 4 | B | A | A |
| Example 6 | Example composition 6 | 5 | 20 | 80 | 150 | 3 | c | A | A |
| Example 7 | Example composition 7 | 5 | 20 | 80 | 150 | 4 | A | A | C |
| Example 8 | Example composition 8 | 5 | 20 | 80 | 150 | 4 | A | B | C |
| Example 9 | Example composition 9 | 5 | 20 | 80 | 150 | 3 | A | B | C |
| Example 10 | Example composition 10 | 5 | 20 | 80 | 150 | 5 | A | A | B |
| Example 11 | Example composition 11 | 5 | 20 | 80 | 150 | 5 | A | A | B |
| Example 12 | Example composition 12 | 5 | 20 | 80 | 150 | 4 | A | A | B |
| Example 13 | Example composition 13 | 5 | 20 | 80 | 150 | 4 | A | A | B |
| Example 14 | Example composition 1 | 0.1 | 20 | 80 | 150 | 5 | A (the same as in Example 1) | A (the same as in Example 1) | B |
| Example 15 | Example composition 1 | 10 | 20 | 80 | 150 | 4 | A (the same as in Example 1) | A (the same as in Example 1) | A |
| Comparative Example 1 | Comparative composition 1 | 5 | 20 | 80 | 150 | 2 | D | A | A |
| Comparative Example 2 | Comparative composition 2 | 5 | 20 | 80 | 150 | 1 | E | A | A |
| Comparative Example 3 | Comparative composition 3 | 5 | 20 | 80 | 150 | 2 | A | C | D |

### [Test for evaluation]

### <Storage stability (-20°C)>

Each of the photopolymerization initiator compositions was sealed into a transparent sample tube and stored for 7 days in a constant-temperature tank with a temperature of -20°C. Then, the way the precipitate occurred was evaluated by visual observation. The evaluation standards are as below. The evaluation results are shown in the second table.
A: the composition was not precipitated.
B: the composition was slightly precipitated.
C: about half of the composition was precipitated.
D: practically the entirety of the composition was precipitated, but a portion of the composition was not precipitated.
E: the entirety of the composition was precipitated.

### <Solubility in water>

0.5 g of each of the photopolymerization initiator compositions was weighed out, and water was added thereto such that the mass thereof increased up to 10 g, thereby preparing a 5% by mass aqueous solution. Then, by using an integrating sphere-type turbidimeter PT-200 (manufactured by Mitsubishi Chemical Analytech Co., Ltd.) and a 10 mm measurement cell and using ultrapure water to set the value of turbidity to zero, the turbidity of the composition was measured. The lower the value of turbidity, the better the solubility of the photopolymerization initiator composition in water.

The evaluation standards are as below. The evaluation results are shown in the second table.
A: less than 25 ppm
B: equal to or higher than 25 ppm and less than 100 ppm
C: equal to or higher than 100 ppm

### <Sensitivity>

A glass plate (trade name "EAGLE XG", manufactured by Corning Incorporated, thickness: 1.1 mm) was spin-coated with each of the active energy ray-curable compositions, and the coating film was dried for 1 minute by using a hot air drier with a temperature of 120°C. Then, by using an ultraviolet exposure device (manufactured by ORC MANUFACTURING CO., LTD., HMW-680GW model), the coating film was irradiated with ultraviolet rays. At this time, the time taken until the coating film became non-sticky in a case where the surface of the coating film was touched with hands was checked, and according to the following evaluation standards, the sensitivity was evaluated. The adhesion of a composition having high sensitivity decreases (the composition having high sensitivity does not feel sticky) even if the composition is irradiated for only a short time. Therefore, such a composition is excellent as a material. The evaluation results are shown in the second table.
A: shorter than 15 seconds.
B: equal to or longer than 15 seconds and shorter than 20 seconds.
C: equal to or longer than 20 seconds and shorter than 30 seconds.
D: equal to or longer than 30 seconds.

### <Evaluation of bleed out after thermocycling>

### (Thermocycling test)

The coating film (cured film) cured after being irradiated with ultraviolet rays in the evaluation of sensitivity was used as a sample and evaluated before and after the thermocycling test. In each cycle of the thermocycling test, the test was performed under a condition 1 and a condition 2 in this order, and the cycle was repeated four times.

(Condition 1) the cured film was stored for 2 weeks in an environment with a temperature of 60°C.

(Condition 2) the cured film was stored for 2 weeks in an environment with a temperature of -20°C.

### (Evaluation of bleed out)

Before and after the thermocycling test, the surface of the cured film of each of the examples and the comparative examples was observed with a microscope (magnification: 200X) so as to observe the bleed out occurring on the cured film. The results were evaluated by being rated at five levels based on the standards shown below. The evaluation results are shown in the second table.
5: no bleed out was observed.
4: extremely slight bleed out was observed, but it was unproblematic for practical use.
3: bleed out was observed, but it was unproblematic for practical use.
2: bleed out occurred, and the cured film could not be used depending on the use.
1: bleed out markedly occurred, and the cured film was unusable for practical use.

### <Evaluation result>

As shown in the second table, in a case where the photopolymerization initiator compositions (example compositions 1 to 13) containing the photopolymerization initiator represented by General Formula (I) and at least one kind of additive selected from the group consisting of compounds represented by General Formulae (II) to (IV) was used, excellent storage stability was exhibited at a low temperature, and excellent solubility in water was maintained (Examples 1 to 15).

Furthermore, as shown in the second table, in a case where active energy ray-curable compositions of examples containing one of the example compositions 1 to 13 as a photopolymerization initiator composition are used, excellent sensitivity could be maintained, and the effect of inhibiting the bleed out occurring on the cured films (cured products) obtained by curing the active energy ray-curable compositions became excellent (Examples 1 to 15).

By comparing Examples 1 to 2 and 10 to 13 with one another, it was revealed that in a case where the specific additive contained in the photopolymerization initiator composition includes only the compounds (General Formulae (III) and (IV)) having a dimeric skeleton (Example 1 (example composition 1), Example 10 (example composition 10), and Example 11 (example composition 11)), the effect of inhibiting the bleed out occurring on the cured film obtained using the active energy ray-curable composition containing the photopolymerization initiator composition is further improved. Particularly, it was revealed that in a case where the specific additive contained in the photopolymerization initiator composition includes only the compound represented by General Formula (IV) having a branched structure, the sensitivity of the active energy ray-curable composition containing the photopolymerization initiator composition is further improved.

By comparing Examples 1 and 7 to 9 with one another, it was revealed that in a case where n (the number of oxyethylene groups as repeating units) in the formulae representing the photopolymerization initiator and the specific additive is 1 (Example 1), the sensitivity of the active energy ray-curable composition is further improved, and the effect of inhibiting the bleed out occurring on the cured film (cured product) is further improved.

By comparing Example 3 with Example 4, it was revealed that in a case where the content of the specific additive with respect to 100 parts by mass of the photopolymerization initiator composition is equal to or smaller than 8 parts by mass (Example 4 (example composition 4)), the sensitivity of the active energy ray-curable composition containing the photopolymerization initiator composition is further improved.

By comparing Example 5 with Example 6, it was revealed that in a case where the content of the specific additive with respect to 100 parts by mass of the photopolymerization initiator composition is equal to or greater than 0.001 parts by mass (Example 5 (example composition 5)), the storage stability of the photopolymerization initiator composition at a low temperature is further improved, and the effect of inhibiting the bleed out occurring on the cured film obtained using the active energy ray-curable composition containing the photopolymerization initiator composition is further improved.

In contrast, it was revealed that in a case where a photopolymerization initiator composition (comparative composition 1) is used which does not contain at least one kind of additive selected from the group consisting of compounds represented by General Formula (II) to (IV), it is impossible to inhibit the bleed out of the cured film obtained using an active energy ray-curable composition containing the photopolymerization initiator composition (Comparative Example 1).

Furthermore, it was revealed that in a case where a photopolymerization initiator composition (comparative composition 2) is used which contains a photopolymerization initiator in which n in General Formula (I) is less than 1, the storage stability at a low temperature deteriorates, and it is impossible to inhibit the bleed out occurring on the cured film obtained using an active energy ray-curable composition containing the photopolymerization initiator composition (Comparative Example 2).

In addition, it was revealed that in a case where a photopolymerization initiator composition (comparative composition 3) is used which contains a photopolymerization initiator in which n in General Formula (I) is greater than 5, the solubility in water deteriorates, and it is impossible to inhibit the bleed out occurring on the cured film obtained using an active energy ray-curable composition containing the photopolymerization initiator composition (Comparative Example 3). It was also revealed that the sensitivity of the active energy ray-curable composition is insufficient.

## Claims

1. A photopolymerization initiator composition comprising:
a photopolymerization initiator represented by General Formula (I); and
at least one kind of additive selected from the group consisting of compounds represented by General Formulae (II) to (IV), 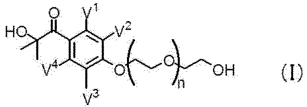 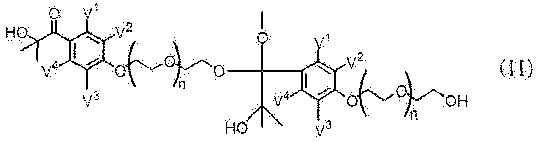 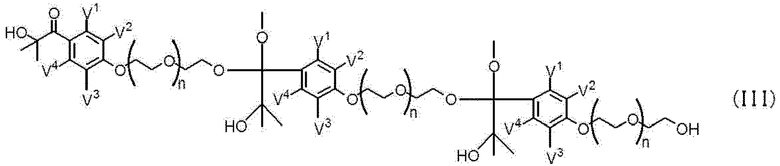 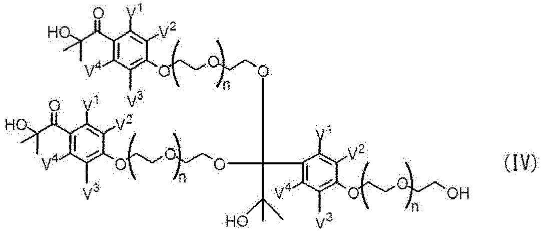
in General Formulae (I) to (IV), V¹, V², V³, and V⁴ each independently represent a hydrogen atom or a substituent, selected from a halogen atom, an alkyl group having 1 to 6 carbon atoms, an alkoxy group having 1 to 6 carbon atoms, a hydroxyl group, an alkylthio group having 1 to 6 carbon atoms, a mercapto group, an acyl group having 1 to 6 carbon atoms or an amino group, and
in General Formula (I) to (IV), n represents an integer of 1 to 5.

2. The photopolymerization initiator composition according to claim 1,
wherein n in General Formulae (I) to (IV) is 1.

3. The photopolymerization initiator composition according to claim 1 or 2,
wherein a content of the additive with respect to 100 parts by mass of the photopolymerization initiator is 0.0005 to 10 parts by mass.

4. The photopolymerization initiator composition according to any one of claims 1 to 3,
wherein the additive is at least one kind of compound selected from the group consisting of compounds represented by General Formula (III) and General Formula (IV).

5. An active energy ray-curable composition comprising:
the photopolymerization initiator composition according to any one of claims 1 to 4; and
a polymerizable compound.

6. The active energy ray-curable composition according to claim 5,
wherein a content of the photopolymerization initiator composition with respect to 100 parts by mass of the polymerizable compound is 0.1 to 10 parts by mass.

7. A cured product obtained by curing the active energy ray-curable composition according to claim 5 or 6.

## Patentansprüche

1. Fotopolymerisationsinitiatorzusammensetzung, enthaltend:
einen Fotopolymerisationsinitiator mit der allgemeinen Formel (I) und
zumindest eine Art von Additiv, ausgewählt aus der Gruppe, bestehend aus Verbindungen mit den allgemeinen Formeln (II) bis (IV): 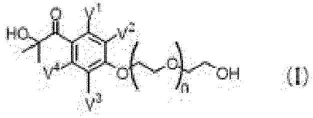 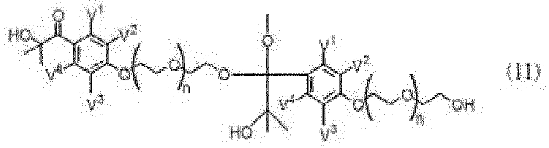 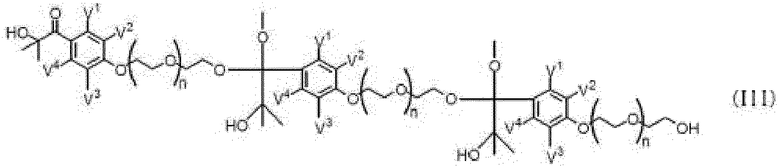 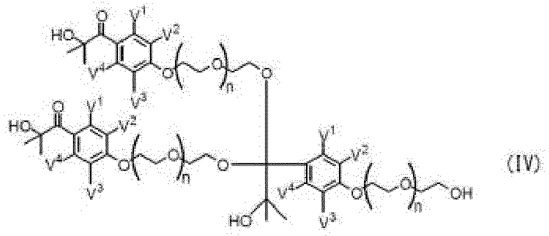 worin in den allgemeinen Formeln (I) bis (IV), V¹, V², V³ und V⁴ jeweils unabhängig ein Wasserstoffatom oder
ein Substituent sind, ausgewählt aus einem Halogenatom einer Alkylgruppe mit 1 bis 6 Kohlenstoffatomen, Alkoxygruppe mit 1 bis 6 Kohlenstoffatomen, Hydroxylgruppe, Alkylthiogruppe mit 1 bis 6 Kohlenstoffatomen, Mercaptogruppe, Acylgruppe mit 1 bis 6 Kohlenstoffatomen oder Aminogruppe und
in der allgemeinen Formel (I) bis (IV) n eine ganze Zahl von 1 bis 5 ist.

2. Fotopolymerisationsinitiatorzusammensetzung gemäß Anspruch 1, worin n in den allgemeinen Formeln (I) bis (IV) 1 ist.

3. Fotopolymerisationsinitiatorzusammensetzung gemäß Anspruch 1 oder 2, worin ein Gehalt des Additivs in Bezug auf 100 Massenteile des Fotopolymerisationsinitiators 0,0005 bis 10 Massenteile ist.

4. Fotopolymerisationsinitiatorzusammensetzung gemäß einem der Ansprüche 1 bis 3, worin das Additiv zumindest eine Art von Verbindung ist, ausgewählt aus der Gruppe, bestehend aus Verbindungen mit der allgemeinen Formel (III) und der allgemeinen Formel (IV).

5. Durch aktive Energiestrahlen härtbare Zusammensetzung, enthaltend:
die Fotopolymerisationsinitiatorzusammensetzung gemäß einem der Ansprüche 1 bis 4 und
eine polymerisierbare Verbindung.

6. Durch aktive Energiestrahlen härtbare Zusammensetzung gemäß Anspruch 5, worin ein Gehalt der Fotopolymerisationsinitiatorzusammensetzung in Bezug auf 100 Masseteile der polymerisierbaren Verbindung 0,1 bis 10 Masseteile ist.

7. Gehärtetes Produkt, erhalten durch Härten der durch aktive Energiestrahlen härtbaren Zusammensetzung gemäß Anspruch 5 oder 6.

## Revendications

1. Composition d'initiateur de photopolymérisation comprenant :
un initiateur de photopolymérisation représenté par la formule générale (I) ; et
au moins un type d'additif sélectionné dans le groupe constitué de composés représentés par les formules générales (II) à (IV), 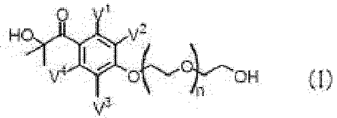 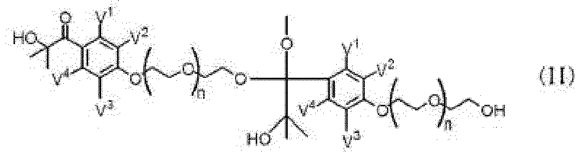 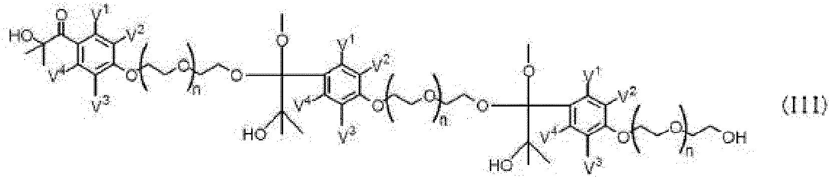 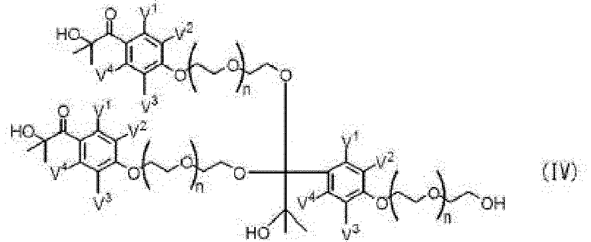
dans les formules générales (I) à (IV), V¹, V², V³, et V⁴ représentent chacun indépendamment un atome d'hydrogène ou un substituant, sélectionné parmi un atome d'halogène, un groupe alkyle ayant 1 à 6 atomes de carbone, un groupe alcoxy ayant 1 à 6 atomes de carbone, un groupe hydroxyle, un groupe alkylthio ayant 1 à 6 atomes de carbone, un groupe mercapto, un groupe acyle ayant 1 à 6 atomes de carbone ou un groupe amino, et
dans les formules générales (I) à (IV),
n représente un nombre entier allant de 1 à 5.

2. Composition d'initiateur de photopolymérisation selon la revendication 1,
dans laquelle n dans les formules générales (I) à (IV) est 1.

3. Composition d'initiateur de photopolymérisation selon la revendication 1 ou 2,
dans laquelle une teneur de l'additif par rapport à 100 parties en masse de l'initiateur de photopolymérisation est de 0,0005 à 10 parties en masse.

4. Composition d'initiateur de photopolymérisation selon l'une quelconque des revendications 1 à 3,
dans laquelle l'additif est au moins un type de composé sélectionné dans le groupe constitué de composés représentés par la formule générale (III) et la formule générale (IV).

5. Composition durcissable sous un rayonnement énergétique actif comprenant :
la composition d'initiateur de photopolymérisation selon l'une quelconque des revendications 1 à 4 ; et
un composé polymérisable.

6. Composition durcissable sous un rayonnement énergétique actif selon la revendication 5,
dans laquelle une teneur de la composition d'initiateur de photopolymérisation par rapport à 100 parties en masse du composé polymérisable est de 0,1 à 10 parties en masse.

7. Produit durci obtenu par durcissement de la composition durcissable sous un rayonnement énergétique actif selon la revendication 5 ou 6.
